Europäisches Patentamt

(19) European Patent Office

Office européen des brevets

(11) Numéro de publication: **0 187 102**
**B1**

(12) **FASCICULE DE BREVET EUROPÉEN**

(45) Date de publication du fascicule du brevet:
**10.08.88**

(21) Numéro de dépôt: **85402630.9**

(22) Date de dépôt: **24.12.85**

(51) Int. Cl.⁴: **C 07 C 9/04,** C 07 C 1/04,
B 01 J 23/28, B 01 J 23/88

(54) **Procédé de production du méthane à l'aide d'un catalyseur thiorésistant et catalyseur pour la mise en oeuvre de ce procédé.**

(30) Priorité: **28.12.84 FR 8420068**

(43) Date de publication de la demande:
**09.07.86 Bulletin 86/28**

(45) Mention de la délivrance du brevet:
**10.08.88 Bulletin 88/32**

(84) Etats contractants désignés:
**AT BE CH DE GB IT LI LU NL SE**

(56) Documents cité:
**FR-A-2 313 332**
**GB-A-2 065 490**

(73) Titulaire: **GAZ DE FRANCE, 23, rue Philibert Delorme, F-75017 Paris (FR)**

(72) Inventeur: **Przydrozny, Michel, 32 rue André Musager, F-95630 Meriel (FR)**
Inventeur: **D'Emmerez de Charmoy, Roger Dennisson, 7 Impasse St. Maur, F-94700 Maisons-Alfort (FR)**
Inventeur: **Sauvion, Guy Noel, 8 rue du Béarn, F-94550 Chevilly- Larue (FR)**
Inventeur: **Caillod, Jack, 31 rue Phanie Leleu, F-95150 Taverny (FR)**

(74) Mandataire: **Durand, Yves Armand Louis, Cabinet Z. Weinstein 20, Avenue de Friedland, F-75008 Paris (FR)**

# 0 187 102

**Descri___ __n**

La présente invention a essentiellement pour objet un procédé de production du méthane ou d'un mélange de gaz comprenant du méthane à l'aide d'un catalyseur résistant au soufre ou thiorésistant.

Elle vise également le catalyseur pour la mise en oeuvre de ce procédé.

Il est déjà connu de réaliser la synthèse du méthane à partir d'un mélange de gaz comprenant entre autres du monoxyde de carbone, de l'hydrogène, de l'eau et des composés soufrés, ladit mélange de gaz pouvant en particulier être obtenu par gazéification du charbon et étant mis en contact avec un catalyseur susceptible d'activer la réaction de synthèse.

Dans ce but, divers catalyseurs ont été proposés, mais on a constaté qu'ils s'usent ou se désactivent rapidement en raison de la présence des composés soufrés.

C'est ainsi que l'on a déjà proposé des catalyseurs pour la réaction de méthanisation qui sont à base par exemple de molybdène et/ou de nickel déposés sur un support d'alumine, mais ces catalyseurs sont loin d'être très satisfaisants sur le plan de leur activité et de leur selectivité en faveur de la production du méthane.

Le document GB-A-2 065 490 décrit un procédé pour produire du méthane dans lequel on utilise un catalyseur qui peut être formé par dépôt par imprégnation de métal (V et/ou Mo) et éventuellement un promoteur d'activité (métal alcalin ou alcalino-terreux) sur un support d'oxyde de titane, d'oxyde de zirconium ou d'hafnium. L'oxyde de titane est utilisé de préférence car il conduit à une activité catalytique supérieure. Aucun exemple ne décrit l'utilisation d'oxyde de zirconium. Les oxydes permettent d'augmenter l'activité du catalyseur et les promoteurs améliorent la sélectivité pour la formation de méthane.

Cependant, il est souhaitable d'avoir à sa disposition d'autres catalyseurs qui permettent d'améliorer la production du méthane et d'obtenir d'excellents résultats tant sur le plan de la conversion du monoxyde de carbone que sur celui de la sélectivité en méthane produit et de la stabilité des performances du catalyseur au cours du temps.

A cet effet, l'invention a pour objet un procédé de production du méthane ou d'un mélange de gaz comprenant du methane, à partir d'un mélange de départ comprenant notamment du monoxyde de carbone, de l'hydrogène, et des composés soufrés, consistant à mettre ledit mélange de départ en contact avec un catalyseur thiorésistant à une température comprise entre 250 et 650°C et à une pression comprise entre 5 et 140 bars, caractérisé en ce qu'on utilise comme catalyseur thiorésistant, un catalyseur comprenant un métal choisi parmi le molybdène, le vanadium et le tungstène et un promoteur d'activité constitué par du cobalt et/ou du nickel lequel catalyseur est déposé par imprégnation sur un support d'oxyde de zirconium, le rapport atomique du métal molybdène, vanadium ou tungstène au zirconium étant compris entre 1/50 et 1/4.

Comme on le verra plus loin, la sélectivité en méthane produit par ce procédé est très supérieure à celle obtenue avec les procédés antérieurs, en raison du fait essentiel que l'on utilise, selon la présente invention, un catalyseur déposé sur oxyde de zirconium.

Suivant une autre caractéristique du procédé selon l'invention, la réaction est effectuée à une vitesse spatiale (V.V.h.) comprise entre 100 et 15 000 heures$^{-1}$ et pour un rapport molaire hydrogène/monoxyde de carbone égal à au moins 0,3 et de préférence égal à 1.

Suivant un mode de réalisation préféré, la réaction est effectuée à une vitesse spatiale égale à 4 750 heures$^{-1}$ et pour un rapport molaire hydrogène/monoxyde de carbone égal à 1, à une pression égale à 30 bars et à une température comprise entre 300 et 600°C et de préférence égale à 500°C.

L'invention vise également un catalyseur pour la mise en oeuvre du procédé répondant à l'une ou l'autre des caractéristiques ci-dessus, ce catalyseur étant caractérisé en ce qu'il est représenté par la formule $X/Co/ZrO_2$, $X/Ni/ZrO_2$ ou $X/Co/Ni/ZrO_2$ dans laquelle X est le molybdène, le vanadium ou le tungstène et en ce qu'il est défini par les caractéristiques suivantes:

- surface spécifique BET du support de $ZrO_2$ supérieure à 10 m$^2$/g,
- volume poreux total compris entre 0,15 et 0,5 cm$^3$/g,
- densité de remplissage tassé comprise entre 0,5 et 2,5,
- rapport atomique du métal X précité au zirconium compris entre 1/50 et 1/4, et
- rapport atomique du métal promoteur d'activité (cobalt et/ou nickel) au métal X compris entre 0,1 et 1 et de préférence entre 0,1 et 0,5.

Selon un mode de réalisation préféré, la surface spécifique BET du catalyseur ci-dessus est égale à 50 m$^2$/g, son volume poreux total est compris entre 0,3 et 0,4 cm$^3$/g, la densité de remplissage tassée est comprise entre 1 et 2, le rapport atomique X/zirconium est compris entre 1/20 et 1/7, et le rapport atomique métal promoteur/X est compris entre 0,1 et 0,5.

Ce catalyseur peut être préparé par les techniques courantes et en particulier par imprégnation du support par des solutions des précurseurs des métaux que l'on désire introduire.

Mais d'autres caractéristiques et avantages de l'invention apparaîtront mieux dans l'exemple ci-après.

On a tout d'abord préparé trois catalyseurs déposés sur oxyde de zirconium, et à savoir: un catalyseur molybdène/oxyde de zirconium, un catalyseur cobalt/ molybdène/oxyde de zirconium et un catalyseur nickel/ molybdène/oxyde de zirconium.

Le catalyseur Mo/ZrO$_2$ présentait une surface spécifique BET de 42 m$^2$/g, un volume poreux total de 0,14 cm$^3$/g, une densité de remplissage tassé de 1,83, et une teneur en molybdène de 3,6 % en poids.

Le catalyseur Co/Mo/ZrO$_2$ présentait une surface spécifique BET de 39 m$^2$/g, un volume poreux à l'eau de 0,11 cm$^3$/g et une densité de remplissage tassé de 1,92.

2

Enfin, le catalyseur $Ni/Mo/ZrO_2$ présentait une surface spécifique BET de 37 $m^2/g$, un volume poreux à l'eau de 0,11 $cm^3/g$ et une densité de remplissage tassé de 1,95.

Ensuite, on a procédé à un test catalytique pour comparer les performances des trois catalyseurs ci-dessus avec des catalyseurs connus à base d'alumine et répondant aux formules suivantes: $Mo/Al_2O_3$, $Co/Mo/Al_2O_3$ et $Ni/Mo/Al_2O_3$.

Ce test catalytique, qui avait pour but de comparer les performances de tous les catalyseurs ci-dessus en termes d'activité et de sélectivité dans la préparation du méthane, a consisté à mettre en contact tous ces catalyseurs avec un mélange de réactifs comprenant dans un premier temps (conditions du tableau 1) 37,25 % en volume de CO (monoxyde de carbone), 37,25 % en volume de $H_2$, 25 % en volume de $H_2O$ et 0,5 % en volume de $H_2S$ et dans un deuxième temps (conditions du tableau 2) 49,75 % en volume de CO, 49,75 % en volume de $H_2$ et 0,5 % en volume de $H_2S$.

La réaction a été effectuée à une vitesse spatiale de 4750 $h^{-1}$, sous une pression de 30 bars et une température de 500°C, après que les catalyseurs aient subi un traitement de présulfuration à 350°C pendant 6 heures sous un courant de 10 litres/h d'un mélange de 1,3 % de $H_2S$ dans l'hydrogène.

Après séparation de l'eau par un condenseur, on a analysé les gaz de sortie par chromatographie en phase gazeuse, ce qui a permis de doser notamment CO, $CO_2$, $CH_4$, $C_2H_6$ et $C_3H_8$.

Cette analyse a permis de calculer d'une part le taux de conversion du monoxyde de carbone (%), défini par le rapport:

$$t_{CO} = \frac{\text{moles de CO consommées}}{\text{moles de CO introduites}} \times 100,$$

et d'autre part, la sélectivité en $CH_4$(%), définie par le rapport:

$$S_{CH_4} = \frac{\text{moles de } CH_4 \text{ formées}}{\text{moles de CO consommées}} \times 100$$

Les tableaux 1 et 2 ci-après illustrent les résultats obtenus sur tous les catalyseurs décrits précédemment, avant et après un vieillissement correspondant à 20 heures de fonctionnement ou de travail du catalyseur.

**Tableau 1.**

| Catalyseurs | | avant vieillissement | | après vieillissement | |
|---|---|---|---|---|---|
| | | $t_{CO}$ % | $S_{CH_4}$ % | $t_{CO}$ % | $S_{CH_4}$ % |
| Art antérieur | $Mo/Al_2O_3$ | 61,7 | 15,2 | 57,4 | 12,2 |
| | $Co/Mo/Al_2O_3$ | 62,6 | 24,8 | 61,6 | 19,1 |
| | $Ni/Mo/Al_2O_3$ | 66,7 | 22,3 | 65 | 18,5 |
| | $Mo/ZrO_2$ | 87,9 | 36,3 | 84,0 | 35,6 |
| Invention | $Co/Mo/ZrO_2$ | 89,1 | 38,1 | 86,7 | 36,9 |
| | $Ni/Mo/ZrO_2$ | 89,3 | 37,5 | 86,1 | 36,2 |

**Tableau 2.**

| Catalyseurs | | avant vieillissement | | après vieillissement | |
|---|---|---|---|---|---|
| | | $t_{CO}$% | $S_{CH_4}$ % | $t_{CO}$ % | $S_{CH_4}$ % |
| Art antérieur | $Mo/Al_2O_3$ | 78,3 | 49,9 | 69,4 | 50,0 |
| | $Co/Mo/Al_2O_3$ | 79,1 | 50,0 | 70,9 | 49,5 |
| | $Ni/Mo/Al_2O_3$ | 78,8 | 49,6 | 71,9 | 49,8 |
| | $Mo/ZrO_2$ | 85,9 | 50 | 80,2 | 49,4 |
| Invention | $Co/Mo/ZrO_2$ | 86,9 | 48,5 | 80,9 | 49,2 |
| | $Ni/Mo/ZrO_2$ | 87,2 | 49,7 | 81,0 | 49,5 |

Les résultats donnés dans le tableau 1 mettent clairement en évidence la supériorité des catalyseurs selon l'invention par rapport aux catalyseurs de l'art antérieur pour ce qui est du taux de conversion du monoxyde de cartone, de la sélectivité en méthane produit, et de la stabilité des performances au cours du temps.

Plus précisément, on remarquera que le catalyseur $Mo/ZrO_2$ est supérieur au premier catalyseur de l'art antérieur mentionné dans le tableau 1 ($Mo/Al_2O_3$) et ne contenant pas d'oxyde de zirconium (sélectivité en méthane après vieillissement de 35,6 % au lieu de 12,2 % pour $Mo/Al_2O_3$).

Cette supériorité est également clairement visible en ce qui concerne les catalyseurs $Co/Mo/ZrO_2$ et

Ni/Mo/Z O$_2$, si on les compare notamment aux catalyseurs de Co ou Ni avec molybdène déposés sur alumine. On voit en effet ici qu'on obtient avec ces deux catalyseurs une sélectivité en méthane comprise entre 36 et 37 % après vieillissement, c'est-à-dire une sélectivité qui est environ le double de celle pour Co/Mo/Al$_2$O$_3$ et Ni/Mo/Al$_2$O$_3$. De plus, on obtient des taux de conversion en CO supérieurs à 80 %.

Les résultats donnés dans le tableau 2 (absence d'eau dans le mélange de départ) montrent que l'on obtient des taux de conversion en CO supérieurs à 85 % et nettement meilleurs que ceux obtenus avec les catalyseurs de l'art antérieur. Les résultats sont également meilleurs pour ce qui est de la stabilité des performances au cours du temps.

Mais ici, la sélectivité en méthane produit est sensiblement la même que celle des catalyseurs de l'art antérieur et se situe dans tous les cas au niveau de la limite thermodynamique.

On a donc réalisé suivant l'invention un procédé de méthanisation qui permet de récupérer des quantités importantes de méthane en utilisant un catalyseur très stable à base d'oxyde de zirconium et résistant efficacement aux composés soufrés tels que H$_2$S, COS, CS$_2$, CH$_3$S etc... et dont la teneur peut dépasser 4 % molaire en soufre.

C'est ainsi qu'en remplaçant le molybdène par du vanadium ou du tungstène dans les formules de catalyseurs conformes au principe de l'invention, on obtient des résultats et des performances proches de ceux décrits ci-dessus. Egalement, l'utilisation conjointe de deux métaux promoteurs d'activité (cobalt et nickel), au lieu d'un seul, conduirait à un catalyseur tout aussi actif en faveur de la production de méthane.

L'invention comprend donc tous les équivalents techniques des moyens décrits ainsi que leurs comtinaisons, si celles-ci sont effectuées suivant son esprit.

## Revendications

1. Procédé de production de méthane ou d'un mélange de gaz comprenant du méthane à partir d'un mélange de départ comprenant notamment du monoxyde de carbone, de l'hydrogène et des composés soufrés, consistant à mettre ledit mélange de départ en contact avec un catalyseur thiorésistant, à une température comprise entre environ 250°C et 650°C et à une pression comprise entre 5 et 140 bars, caractérisé en ce qu'on utilise, comme catalyseur thiorésistant, un catalyseur comprenant un métal choisi dans le groupe formé par le molybdène, le vanadium et le tungstène et un promoteur d'activité constitué par du cobalt et/ou du nickel, ce catalyseur étant déposé par imprégnation sur un support d'oxyde de zirconium, le rapport atomique du métal molybdène, vanadium ou tungstène au zirconium étant compris entre 1/50 et 1/4.

2. Procédé selon la revendication 1, caractérisé en ce que la réaction est effectuée à une visesse spatiale comprise entre 100 et 15 000 heures$^{-1}$ et pour un rapport molaire hydrogène/monoxyde de carbone égal à au moins 0,3 et de préférence égal à 1.

3. Procédé selon la revendication 1 ou 2, caractérisé en ce que la réaction est effectuée à une vitesse spatiale égale à 4750 heures$^{-1}$ et pour un rapport molaire hydrogène/monoxyde de carbone égal à 1, à une pression égale à 30 bars et à une température comprise entre 300 et 600°C et de préférence égale à 500°C.

4. Catalyseur pour la mise en oeuvre du procédé selon l'une des revendications 1 à 3, caractérisé en ce qu'il répond à la formule X/Co/ZrO$_2$, X/Ni/ZrO$_2$ ou X/Co/Ni/ZrO$_2$ dans laquelle X est le molybdène, le vanadium ou le tungstène et en ce que ledit catalyseur est défini par les caractéristiques suivantes:
- surface spécifique BET du support de ZrO$_2$ supérieure à 10 m$^2$/g;
- volume poreux total compris entre 0,15 et 0,5 cm$^3$/g;
- densité de remplissage tassé comprise entre 0,5 et 2,5.
- rapport atomique du métal X précité au zirconium compris entre 1/50 et 1/4, et
- rapport atomique du métal promoteur d'activité (Co et/ou Ni) au métal X compris entre 0,1 et 1, et de préférence entre 0,1 et 0,5.

5. Catalyseur selon la revendication 4, caractérisé en ce que la surface spécifique BET précitée est égale à 50 m$^2$/g, le volume poreux total précité est compris entre 0,3 et 0,4 cm$^3$/g, la densité de remplissage tassé est comprise entre 1 et 2, le rapport atomique X/zirconium est compris entre 1/20 et 1/7, et le rapport atomique métal promoteur/X est compris entre 0,1 et 0,5.

## Patentansprüche

1. Verfahren zur Erzeugung von Methan oder von einer methanhaltigen Gasmischung aus einer insbesondere Kohlenmonoxid, Wasserstoff und Schwefelverbindungen enthaltenden Ausgangsmischung, das darin besteht, die besagte Ausgangsmischung bei einer zwischen ca. 250°C und 650°C liegenden Temperatur und unter einem zwischen 5 und 140 bar liegenden Druck mit einem schwefelfesten Katalysator in Kontakt zu bringen, dadurch gekennzeichnet, daß man als schwefelfesten Katalysator einen Katalysator verwendet, der ein in der von Molybdän, Vanadium und Wolfram gebildeten Gruppe gewähltes Metall und einen aus Kobalt und/oder Nickel bestehenden Aktivitätspromotor enthält, wobei dieser Katalysator durch Imprägnierung auf einen Zirkoniumoxidträger absetzt ist und das Atomverhältnis vom Molybdän, Vanadium oder Wolframmetall zum Zirkonium zwischen 1/50 und 1/4 liegt.

2. Verfahren nach Anspruch 1, dadurch gekennzeichnet, daß die Reaktion bei einer zwischen 100 und 15 000 Stunden[-1] liegenden Raumgeschwindigkeit und bei einem wenigstens 0,3 und vorzugsweise 1 betragenden Wasserstoff/Kohlenmonoxid-Molverhältnis durchgeführt wird.

3. Verfahren nach Anspruch 1 oder 2, dadurch gekennzeichnet, daß die Reaktion bei einer 4 750 Stunden[-1] betragenden Raumgeschwindigkeit und bei einem 1 betragenden Wasserstoff/Kohlenmonoxid-Molverhältnis, unter einem 30 bar betragenden Druck und bei einer zwischen 300 und 600°C und vorzugsweise 500°C betragenden Temperatur durchgeführt wird.

4. Katalysator zur Durchführung des Verfahrens nach einem der Ansprüche 1 bis 3, dadurch gekennzeichnet, daß er der Formel $X/Co/ZrO_2$, $X/Ni/ZrO_2$ oder $X/Co/Ni/ZrO_2$ worin X für Molybdän, Vanadium oder Wolfram steht, entspricht und daß der besagte Katalysator durch die folgenden Merkmale bestimmt ist:
- BET-spezifische Oberfläche des $ZrO_2$-Träger größer als 10 $m^2/g$.
- gesamtes Porenvolumen zwischen 0,15 ud 0,5 $cm^3/g$;
- gepackte Füllungsdichte zwischen 0,5 und 2,5;
- Atomverhältnis des besagten Metall X zum Zirkonium zwischen 1/50 und 1/4, und
- Atomverhältnis des metallischen Aktivitätspromotors (Co und/oder Ni) zum Metall X zwischen 0,1 und 1, vorzugsweise zwischen 0,1 und 0,5.

5. Katalysator nach Anspruch 4, dadurch gekennzeichnet, daß die besagte BET-spezifische Oberfläche 50 $m^2/g$ beträgt, daß das besagte gesamte Porenvolumen zwischen 0,3 und 0,4 $cm^3/g$ liegt, daß die gepackte Füllungsdichte zwischen 1 und 2 liegt, daß das Atomverhältnis X/Zirkonium zwischen 1/20 und 1/7 liegt, und daß das Atomverhältnis metallischer Promotor/X zwischen 0,1 und 0,5 liegt.

## Claims

1. Method of production of methane or of a methane-containing gas mixture from an initial mixture comprising in particular carbon monoxide, hydrogen and sulphur compounds, which consists in contacting the said initial mixture with a thioresistant catalyst, at a temperature lying between about 250°C and 650°C and at a pressure lying between 5 and 140 bars, characterized in that use is made, as a thioresistant catalyst, of a catalyst comprising a metal selected from the group comprising molybdenum, vanadium and tungsten and an activity promoter consisting of cobalt and/or nickel, which catalyst being deposited through impregnation onto a zirconium oxide carrier, the atomic ratio of the molybdenum, vanadium or tungsten metal to zirconium lying between 1/50 and 1/4.

2. Method according to claim 1, characterized in that the reaction is performed at a space velocity lying between 100 and 15 000 hours[-1] and with a hydrogen/carbon monoxide molar ratio equal to at least 0.3 and preferably equal to 1.

3. Method according to claim 1 or 2, characterized in that the reaction is carried out at a space velocity equal to 4750 hours[-1] and with a hydrogen/carbon monoxide molar ratio equal to 1, at a pressure equal to 30 bars and at a temperature lying between 300 ad 600°C and preferably equal to 500°C.

4. Catalyst for carrying out the method according to one of claims 1 to 3, characterized in that it meets the formula $X/Co/ZrO_2$, $X/Ni/ZrO_2$ or $X/Co/Ni/ZrO_2$ wherein X stands for molybdenum, vanadium or tungsten and in that said catalyst is defined by the following characteristics:
- a BET specific area of the $ZrO_2$ carrier above 10 $m^2/g$;
- a total porous volume lying between 0.15 $cm^3/g$ and 0.5 $cm^3$;
- a packed filling density lying between 0.5 and 2.5;
- an atomic ratio of said X metal to zirconium lying between 1/50 and 1/4, and
- an atomic ratio of the activity promoter metal (Co and/or Ni) to the X metal lying between 0.1 and 1, and preferably between 0.1 and 0.5.

5. Catalyst according to claim 4, characterized in that said BET specific area is equal to 50 $m^2/g$, said total porous volume is lying between 0.3 and 0.4 $cm^3/g$, the packed filling density is lying between 1 and 2, the X/zirconium atomic ratio is lying between 1/2 and 1/7 and the promoter metal/X atomic ratio is lying between 0.1 and 0.5.